# EUROPEAN PATENT SPECIFICATION

(11) **EP 3 621 621 B1**
(45) Date of publication and mention of the grant of the patent: **25.12.2024**
(21) Application number: 18798779.7
(22) Date of filing: 10.05.2018
(51) Int. Cl.: A61K 9/24, A61K 9/20, A61K 9/14, A61K 31/4196, A61K 31/4045, A61K 31/5415, A61K 47/02, A61K 47/40, A61K 47/69, A61P 29/00, C08B 37/16, C08L 5/16

(54) **PHARMACEUTICAL COMPOSITIONS COMPRISING MELOXICAM**
PHARMAZEUTISCHE ZUSAMMENSETZUNGEN MIT MELOXICAM
COMPOSITIONS PHARMACEUTIQUES CONTENANT DU MÉLOXICAM

(30) Priority: 10.05.2017 US 201762504105 P; 29.06.2017 US 201762526884 P; 25.07.2017 US 201762536466 P; 04.01.2018 WO PCT/US2018/012433
(43) Date of publication of application: 18.03.2020
(60) Divisional application: 24206002.8
(73) Proprietor: Axsome Therapeutics, Inc., New York, NY 10007 (US)
(72) Inventor: TABUTEAU, Herriot, New York, NY 10007 (US)
(74) Representative: Murgitroyd & Company
(86) International application number: PCT/US2018/032162
(87) International publication number: WO 2018/209150

(56) References cited:
- WO-A1-00/25779
- WO-A1-2017/066488
- WO-A1-2017/066488
- WO-A1-2018/129220
- WO-A2-2016/131067
- US-A- 6 046 177
- US-A1- 2006 105 045
- US-A1- 2008 103 189
- US-A1- 2016 228 576
- US-A1- 2016 228 576
- US-B1- 6 284 269

## Description

### BACKGROUND

There continues to be a need for therapies with improved efficacy in treating pain, inflammation, and related conditions.

US 2008/0103189 relates to substituted indoles for use in treating various medical disorders. US 2016/0228576 and US 6,284,269 relate to pharmaceutical compositions comprising meloxicam, a cyclodextrin and/or carbonate/bicarbonate. US 2006/0105045 relates to the use of cyclodextrins or cyclodextrin derivatives as solubility enhancers when formulated in water-miscible organic solvents. US 6,046,177 relates to a tablet core made from granules comprising SBE₇-β cyclodextrin and methylprednisolone dry blended with sodium bicarbonate. WO 2017/066488 relates to compositions comprising an opioid and an antiemetic for treating pain. WO 00/25779 relates to a pharmaceutical compositions comprising a 5HT_{1B/1D} agonist and a COX-2 selective inhibitor for treating migraine.

### SUMMARY

A first aspect of the invention provides a solid oral dosage form comprising: an inclusion complex of meloxicam and a sulfobutyl ether β-cyclodextrin (SBEβCD), wherein the solid dosage form contains about 20 mg of the meloxicam or a molar equivalent amount of a salt thereof; a bicarbonate; and about 10 mg of rizatriptan or a molar equivalent amount of a salt thereof.

In some embodiments, the dosage form has a shorter Tₘₐₓ of meloxicam than a reference dosage form that: 1) contains the same amount of meloxicam, 2) does not contain an SBEβCD, and 3) does not contain a bicarbonate, when directly administered orally to the mammal.

In some embodiments, the solid oral dosage form comprises an inclusion complex of: 1) the rizatriptan; and 2) the SBEβCD.

In some embodiments, the SBEβCD has 6 to 7 sulfobutyl ether groups for each molecule of β-cyclodextrin.

In some embodiments, the solid oral dosage form contains 50 mg to 200 mg of the SBEβCD.

In some embodiments, the solid oral dosage form contains 100 mg of the SBEβCD.

In some embodiments, the bicarbonate comprises sodium bicarbonate.

In some embodiments, the solid oral dosage form contains 400 mg to 600 mg of the bicarbonate.

In some embodiments, the solid oral dosage form contains 500 mg of the bicarbonate.

In some embodiments, the solid oral dosage form has been shown to have a mean Tₘₐₓ of meloxicam that is less than about 2 hours.

In some embodiments, the solid oral dosage form has increased bioavailability of the meloxicam as compared to the reference dosage form when administered to a mammal.

In some embodiments, the solid oral dosage form has increased bioavailability of the rizatriptan as compared to the reference dosage form when administered to a mammal.

A second aspect of the invention provides the solid dosage form of the invention for use in the treatment of pain in a mammal in need thereof.

In some embodiments, the pain is migraine.

In some embodiments, the pain is inflammatory pain

### BRIEF DESCRIPTION OF THE DRAWINGS

Figure 1 is a depiction of the results described in Example 2 and contained in Table 6.
Figure 2 is another depiction of the results described in Example 2 and contained in Table 6.
Figure 3 is another depiction of the results described in Example 2 and contained in Table 6.
Figure 4 is another depiction of the results described in Example 2 and contained in Table 6.
Figure 5 is another depiction of the results described in Example 2 and contained in Table 6.
Figure 6 is another depiction of the results described in Example 2 and contained in Table 6.
Figure 7 is another depiction of the results described in Example 2 and contained in Table 6.
Figure 8 is another depiction of the results described in Example 2 and contained in Table 6.
Figure 9 is another depiction of the results described in Example 2 and contained in Table 6.
Figure 10 is another depiction of the results described in Example 2 and contained in Table 6.
Figure 11 is a plot of meloxicam plasma concentration at various time points over the first 24 hours for an embodiment of a dosage form described herein and a commercially available meloxicam dosage form.

### DETAILED DESCRIPTION

Meloxicam and some other NSAIDs, and other drugs, have poor aqueous solubility which may reduce bioavailability and slow the onset of pain relief. One method of increasing the solubility and bioavailability of meloxicam or another drug is through the use of cyclodextrins in combination with meloxicam.

The invention provides a solid oral dosage form comprising: an inclusion complex of meloxicam and a sulfobutyl ether β-cyclodextrin (SBEβCD), wherein the solid dosage form contains about 20 mg of the meloxicam or a molar equivalent amount of a salt thereof; a bicarbonate; and about 10 mg of rizatriptan or a molar equivalent amount of a salt thereof. Rizatriptan is a triptan. Other triptans are sumatriptan, naratriptan, eletriptan, donitriptan, almotriptan, frovatriptan, alvitriptan, and zolmitriptan. However, in the invention, the triptan is rizatriptan, which has the structure as shown below.

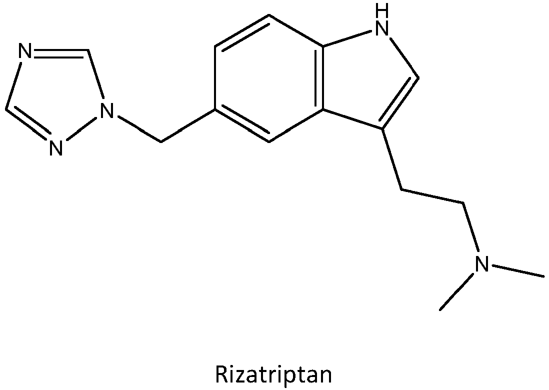

Meloxicam is an NSAID. Other NSAIDs include celecoxib, rofecoxib, lumiracoxib, valdecoxib, parecoxib, etoricoxib, CS-502, JTE-522, L-745,337, NS398, aspirin, acetaminophen (considered to be an NSAID for the purposes of the present disclosure), ibuprofen, flurbiprofen, ketoprofen, naproxen, oxaprozin, etodolac, indomethacin, ketorolac, lornoxicam, meloxicam, piroxicam, droxicam, tenoxicam, nabumetone, diclofenac, meclofenamate, mefenamic acid, diflunisal, sulindac, tolmetin, fenoprofen, suprofen, benoxaprofen, aceclofenac, tolfenamic acid, oxyphenbutazone, azapropazone, phenylbutazone.

However, in the invention, the NSAID is meloxicam, which has the structure:

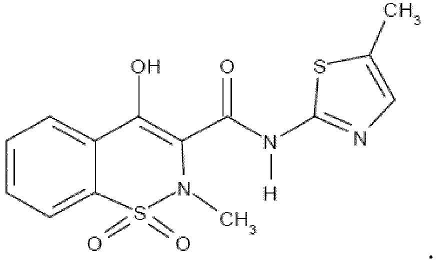

Meloxicam exhibits anti-inflammatory, analgesic, and antipyretic activities. The meloxicam mechanism of action may be related to the inhibition of prostaglandin synthetase (cyclo-oxygenase, COX) which is involved in the initial steps of the arachidonic acid cascade, resulting in the reduced formation of prostaglandins, thromboxanes and prostacylin.

In the invention, the dosage form is given orally.

The term "treating" or "treatment" broadly includes any kind of treatment activity, including the diagnosis, cure, mitigation, or prevention of disease in man or other animals, or any activity that otherwise affects the structure or any function of the body of man or other animals.

The dosage form may be used to treat, or provide relief of, any type of pain including, but not limited to, migraine and other types of headache, inflammatory pain, musculoskeletal pain, neuropathic pain, chronic pain, acute pain, localized pain, systemic pain, cancer-related pain, acute pain, pain due to injury, pain due to illness (e.g., fever), post-operative pain, etc. In some instances, pain relief may be palliative, or pain relief may be provided independent of improvement of the disease or condition or the underlying cause of the disease or condition. For example, although the underlying disease may not improve, or may continue to progress, an individual suffering from the disease may experience pain relief. The pain may affect a muscle, nerve, cartilage, bone, ligament, tendon, tendon sheaths, bursae, or joint.

Migraine is a headache disorder characterized by recurrent headaches that may be moderate to severe. The headaches may affect one half of the head, may be pulsating in nature, and may last from 2 to 72 hours. Associated symptoms may include nausea, vomiting, and sensitivity to light (photophobia), sound (phonophobia), or smell. The pain can be made worse by physical activity. Migraines may be associated with an aura, which may be a short period of visual disturbance which signals that the headache will soon occur.

The dosage form may be administered to relieve inflammatory pain, including inflammatory musculoskeletal pain, pain due to injury, arthritis pain, and complex regional pain syndrome. The inflammatory pain may be chronic or acute.

The dosage form may be administered to relieve arthritis pain, or other signs and/or symptoms of arthritis. Arthritis refers to inflammatory joint diseases that can be associated with pain. Examples of arthritis include, but are not limited to, rheumatoid arthritis, juvenile rheumatoid arthritis (pauciarticular and polyarticular course), osteoarthritis, erosive osteoarthritis, sero-negative (non-rheumatoid), arthropathies, non-articular rheumatism, peri-articular disorders, axial spondyloarthritis, transient osteoarthritis of the hip, vertebral crush fractures, arthritis associated with osteoporosis, and neuropathic arthropathies including Charcot's foot, axial spondyloarthritis including ankylosing spondylitis, and SAPHO syndrome. The arthritis pain may be chronic or acute. The dosage form may be administered to relief the signs and/or symptoms of an arthritis including but not limited to osteoarthritis.

The dosage form may be administered to relieve neuropathic pain, including diabetic peripheral neuropathy, post-herpetic neuralgia, trigeminal neuralgia, monoradiculopathies, phantom limb pain, sciatica, pudendal neuralgia, and central pain. Other causes of neuropathic pain may include, but are not limited to, cancer-related pain, lumbar nerve root compression, spinal cord injury, post-stroke pain, central multiple sclerosis pain, HIV-associated neuropathy, and radio-therapy or chemo-therapy associated neuropathy. The neuropathic pain may be chronic or acute.

The dosage form may be administered to relieve musculoskeletal pain. Examples of musculoskeletal pain may include, but are not limited to, back pain, low back pain (e.g., lumbosacral pain), neck pain, infection, cramps, tendonitis, epidondylitis, carpal tunnel syndrome, joint pain, fibromyalgia, pain due to injury, Tunnel syndromes, pain associated with bone fractures, sprains, fibrous dysplasia, osteogenesis imperfecta, Paget's disease of bone, transient osteoporosis, and transient osteoporosis of the hip. The musculoskeletal pain may be chronic or acute.

Administration of the dosage form may achieve a reduction in pain that lasts at least about one hour, at least about two hours, at least about three hours, at least about four hours, at least about six hours, at least about eight hours, about 8 to about 24 hours, or about 24 hours. Administration of the dosage form may achieve a reduction in pain that is observed at about 10 minutes, at about 30 minutes, at about one hour, at about two hours, at about three hours, at about four hours, at about five hours, at about six hours, at or less than about 5, 10, 15, 20, 25, 30, 35, 40, 45, 50, or 60 minutes, at two hours or less, at three hours or less, or other time period bound by these ranges, after administration of the dosage form.

A human being that is treated for a disease or condition with any of the dosage forms described herein. may be of any age. For example the person may have an age of about 10-90 years, about 20-80 years, about 30-75 years, about 40-70 years, about 1-16 years, about 80-95 years, about 18 years or more, about 20 years or more, about 25 years or more, about 30 years or more, about 40 years or more, about 45 years or more, about 50 years or more, about 55 years or more, about 60 years or more, about 65 years or more, or any other age in a range bounded by, or between, any of these values.

A human being that is treated for a disease or condition with a dosage form may have suffered from the pain or condition associated with the pain for at least 1 day, at least one week, at least 2 weeks, at least 1 month, at least 6 weeks, at least 2 months, at least 3 months, at least 6 months, at least 1 year, at least 5 years, at least 10 years, at least 15 years, at least 20 years, at least 30 years, at least 40 years, at least 50 years or any duration in a range bounded by, or between, any of these values.

In the invention, the meloxicam is in an inclusion complex with a sulfobutyl ether β-cyclodextrin (SBEβCD). A sulfobutyl ether β-cyclodextrin (SBEβCD), which isa cyclodextrin Cyclodextrins (also known as cycloamyloses) are generally cyclic polysaccharides which form a bucket-like shape. Cyclodextrins help to increase bioavailability of other molecules because cyclodextrins are hydrophobic on the inside and hydrophilic on the outside which helps to facilitate the transport of hydrophobic molecules to a hydrophilic medium. The naturally occurring cyclodextrins include six, seven, and eight glucose units (α, β, and γ-cyclodextrin, respectively). However, synthetic cyclodextrins containing more or less glucose units are possible. In aqueous solutions, cyclodextrins can form complexes (i.e., an inclusion complex) with drugs by incorporating the drug into the center/hydrophobic portion of the cyclodextrin ring; although cyclodextrins are also known to aggregate around a drug in a micelle-type structure. This ability of cyclodextrins may allow them to act as carriers of less soluble drugs to increase the drugs' bioavailability.

The inclusion complex of the invention may be more water-soluble relative to the non-complexed drug. The cyclodextrin of the invention is a sulfobutyl ether β-cyclodextrin (SBEβCD). The cyclodextrin Cyclodextrins also include a naturally-occurring cyclodextrin (e.g., α, β, or γ-cyclodextrins) or a synthetic cyclodextrin. α-Cyclodextrins, derivatives, or salts thereof. α-Cyclodextrins may include, but are not limited to, (2,3,6-tri-O-acetyl)-α-cyclodextrin, (2,3,6-tri-O-methyl)-α-cyclodextrin, (2,3,6-tri-O-octyl)-α-cyclodextrin, 6-bromo-6-deoxy-α-cyclodextrin, 6-iodo-6-deoxy-α-cyclodextrin, (6-O-tertbutyl-dimethylsilyl)-α-cyclodextrin, butyl-α-cyclodextrin, succinyl-α-cyclodextrin, (2-hydroxypropyl)-α-cyclodextrin, or combinations thereof. However, the cyclodextrin of the invention is a sulfobutyl ether β-cyclodextrin (SBEβCD).

β-Cyclodextrins, derivatives, or salts thereof that are not part of the invention include, but are not limited to, hydroxypropyl-β-cyclodextrin, 6-monodeoxy-6-monoamino-β-cyclodextrin, glucosyl-β-cyclodextrin, maltosyl-β-cyclodextrin, 6-O-α-D-glucosyl-β-cyclodextrin, 6-O-α-maltosyl-β-cyclodextrin, 6-azido-6-deoxy-β-cyclodextrin, (2,3-di-O-acetyl-6-O-sulfo)-β-cyclodextrin, methyl-β-cyclodextrin, dimethyl-β-cyclodextrin (DMβCD), trimethyl-β-cyclodextrin (TMβCD), (2,3-di-O-methyl-6-O-sulfo)-β-cyclodextrin, (2,6-di-O-methyl)-β-cyclodextrin, (2,6-di-O-ethyl)-β-cyclodextrin, (2,3,6-tri-O-methyl)-β-cyclodextrin, (2,3,6-tri-O-acetyl)-β-cyclodextrin, - (2,3,6-tri-O-benzoyl)-β-cyclodextrin, (2,3,6-tri-O-ethyl)-β-cyclodextrin, 6-iodo-6-deoxy-β-cyclodextrin, 6-(dimethyl-tert-butylsilyl)-6-deoxy-β-cyclodextrin, 6-bromo-6-deoxy-β-cyclodextrin, monoacetyl-β-cyclodextrin, diacetyl-β-cyclodextrin, triacetyl-β-cyclodextrin, (3-O-acetyl-2,6-di-O-methyl)-β-cyclodextrin, (6-O-maltosyl)-β-cyclodextrin, (6-O-sulfo)-β-cyclodextrin, (6-O-t-butyldimethylsilyl-2,3-di-O-acetyl)-β-cyclodextrin, succinyl-(2-hydroxypropyl)-β-cyclodextrin, (2,6-di-O-)ethyl-β-cyclodextrin, (2-carboxyethyl)-β-cyclodextrin (CMEβCD), hydroxyethyl-β-cyclodextrin (HEβCD), (2-hydroxypropyl)-β-cyclodextrin, (2-hydroxypropyl)-β-cyclodextrin (HPβCD), (3-hydroxypropyl)-β-cyclodextrin (3HPβCD), (2,3-hydroxypropyl)-β-cyclodextrin (DHPβCD), butyl-β-cyclodextrin, methyl-β-cyclodextrin, silyl((6-O-tert-butyldimethyl)-2,3,-di-O-acetyl)-β-cyclodextrin, succinyl-β-cyclodextrin, (2-hydroxyisobutyl)-β-cyclodextrin, randomly methylated-β-cyclodextrin, branched-β-cyclodextrin, or combinations thereof.

A β-cyclodextrin may be a sulfoalkyl ether cyclodextrin, derivative, or salt thereof. Examples of sulfoalkyl ether cyclodextrin derivatives may include, but are not limited to, sulfobutyl ether-β-cyclodextrin (e.g., SBEβCD, betadex, CAPTISOL^{®}). The β-cyclodextrin of the invention is a sulfobutyl ether β-cyclodextrin (SBEβCD). A SBEβCD may have about 4-8, about 5-8, about 4-7, about 6-7, or about 6.5 sulfobutyl ether groups per cyclodextrin molecule.

γ-Cyclodextrins, derivatives, or salts thereof that are not part of the invention include carboxymethyl-γ-cyclodextrin, (2,3,6-tri-O-acetyl)-γ-cyclodextrin, (2,3,6-tri-O-methyl)-γ-cyclodextrin, (2,6-di-O-pentyl)-γ-cyclodextrin, 6-(dimethyl-tert-butylsilyl)-6-deoxy-γ-cyclodextrin, 6-bromo-6-deoxy-γ-cyclodextrin, 6-iodo-6-deoxy-γ-cyclodextrin, (6-O-t-butyldimethylsilyl)-γ-cyclodextrin, succinyl-γ-cyclodextrin, hydroxypropyl-γ-cyclodextrin, (2-hydroxypropyl)-γ-cyclodextrin, acetyl-γ-cyclodextrin, butyl-γ-cyclodextrin, or combinations thereof.

The dosage form includes a bicarbonate, such as sodium bicarbonate, potassium bicarbonate, etc. A bicarbonate may help to increase the pharmacokinetics or bioavailability of meloxicam or rizatriptan.

Enhancing bioavailability of meloxicam or rizatriptan in the dosage form may allow a reduced molar amount of the drug to be used as compared to other dosage forms containing the drug in treating diseases or disorders.

Use of the claimed cyclodextrin or bicarbonate may improve the oral bioavailability (e.g. a higher Cₘₐₓ and/or higher AUC) of meloxicam in a subject (human or animal) by at least about 10%, at least about 20%, at least about 30%, at least about 40%, at least about 50%, at least about 60%, at least about 70%, at least about 80%, at least about 90%, up to about 100%, up to about 200%, or any amount in a range bounded by, or between, any of these values as compared to administration of meloxicam alone.

Use of the claimed cyclodextrin or a bicarbonate may improve the oral bioavailability (e.g. a higher Cₘₐₓ and/or higher AUC) of rizatriptan in subject (human or animal) by at least about 10%, at least about 20%, at least about 30%, at least about 40%, at least about 50%, at least about 60%, at least about 70%, at least about 80%, at least about 90%, up to about 100%, up to about 200%, or any amount in a range bounded by, or between, any of these values as compared to administration of the rizatriptan alone.

Due to the improved bioavailability as described above, the dosage form may contain, or a subject may receive, on a molar basis, less of the rizatriptan or meloxicam than would otherwise be administered of the rizatriptan or meloxicam alone. For example, a dosage form may contain, or a mammal may receive, at least about 10 mole% less, at least about 20 mole% less, at least about 30 mole% less, at least about 40 mole% less, at least about 50 mole% less, at least about 60 mole% less, at least about 70 mole% less, at least about 80 mole% less, at least about 85 mole% less, and/or up to about 90 mole% less, 95 mole% less, 98 mole% less, or any amount in a range bounded by, or between, any of these values of meloxicam as that would otherwise be administered of meloxicam alone.

Use of other NSAIDs, opioids, or other pain medications may be reduced by at least about 5%, at least about 10%, at least about 15%, at least about 20%, at least about 25%, at least about 30%, at least about 35%, at least about 40%, at least about 45%, at least about 50%, at least about 60%, at least about 70%, at least about 80%, or at least about 90%, up to about 100%, or any amount in a range bounded by, or between, any of these values when administered with the invention, as compared to administration of the NSAID, the opioid or the other pain medication alone.

A dosage form contains meloxicam in an amount of about 20 mg For the amount of meloxicam described herein, salt forms of meloxicam may be present in the amount recited above, or an amount that is a molar equivalent to this amount for the non-salt form of meloxicam. The claimed dose of meloxicam may be a safe dose for repeated administration, such as 1, 2, 3, or 4 times a day, or repeated at an interval of 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 31 days, about 1-2 months, about 4 weeks, about 6 weeks, about 2-3 months, about 3-4 months, about 4-5 months, about 5-6 months, about 6-7 months, about 7-8 months, about 8-9 months, about 9-10 months, about 10-11 months, about 11-12 months, about 2 years, etc.

Formation of an inclusion complex may help to improve the properties of a dosage form. For some inclusion complexes, the meloxicam and the SBEβCD may have a molar ratio of about 0.5-2 (a molar ratio of 0.5 is 0.5 moles of the drug to 1 mole of cyclodextrin), about 0.5-0.7, about 0.6-0.8, about 0.7-0.9, about 0.8-1, about 0.9-1.1, about 1-1.2, about 1.1-1.3, about 1.2-1.4, about 1.3-1.5, about 1.4-1.6, about 1.5-1.7, about 1.6-1.8, about 1.7-1.9, about 1.8-2, about 1.9-2.1, about 2-2.2, about 0.8-1.2, about 1, or any ratio in a range bounded by any of these values.

An inclusion complex may be formed by (1) mixing a homogeneous solution of meloxicam with a homogeneous solution of the SBEβCD to form a homogeneous solution of the meloxicam and the cyclodextrin, and (2) removing or evaporating the solvent of the homogeneous solution of the meloxicam and the SBEβCD to form the complex comprising the inclusion complex of the meloxicam in a SBEβCD. The solutions can be pH-adjusted aqueous solutions. The pH can be adjusted using a buffering agent. The solvent can be removed or evaporated by lyophilization, spray drying, or any other means that is suitable. The solvent can be removed by vacuum drying, etc.

For some dosage forms, a SBEβCD may be employed in a weight ratio to the meloxicam within the range of about 1-1000 (e.g. 1 g of cyclodextrin per 1 g of meloxicam is a weight ratio of 1); about 1-500, about 1-5, about 1-20; about 1-10; about 1-15; about 2-4, about 3-5, about 4-6, about 5-7, about 6-8, about 7-9, about 8-10, about 0.01-1; about 0.05-1; about 0.1-1; about 0.2-1; about 0.3-1, about 0.4-1, about 0.5-1, about 0.6-1, about 0.7-1, about 0.8-1, or any weight ratio in a range bounded by, or between, any of these values. Each type of cyclodextrin employed may have a different weight ratio to the meloxicam in the dosage form.

For some dosage forms, a SBEβCD may be employed in a weight ratio to rizatriptan within the range of about 1-1000 (e.g. 10 g of SBEβCD per 1 g of rizatriptan is a weight ratio of 10); about 1-500; about 1-100; about 1-50; about 1-20; about 1-10; about 1-15; about 2-4, about 3-5, about 4-6, about 5-7, about 6-8, about 7-9, about 8-10, about 9-11, about 10-12, about 11-13, about 12-14, about 13-15, about 14-16, about 15-17, about 16-18, about 17-19, about 18-20, about 19-21, about 0.001-1; about 0.01-1; about 0.05-1; about 0.1-1; about 0.2-1; about 0.3-1, about 0.4-1, about 0.5-1, about 0.6-1, about 0.7-1, about 0.8-1, or any weight ratio in a range bounded by, or between, any of these values. Each type of SBEβCD employed may have a different weight ratio to rizatriptan in the dosage form.

For the amount of rizatriptan, salt forms of rizatriptan may be present in the amounts recited, or amounts that are molar equivalents to these amounts for the rizatriptan free base. For example, assuming that the molecular weight of rizatriptan free base is 269.3 g/mol, 10 mg of rizatriptan is 37.1 mmol of rizatriptan. Thus, a molar equivalent of 10 mg of rizatriptan free base would be the mass of 37.1 mmol of that salt form. For example, for the benzoate salt (mw = 391.2 g/mol), the molar equivalent of 10 mg of the free base (or 37.1 mmol), would be 14.5 mg. These doses may be a safe dose for repeated administration, such as 1, 2, 3, or 4 times a day, or repeated at an interval of 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 31 days, 4 weeks, 4-6 weeks, about 1-2 months, about 6 weeks, about 2-3 months, about 3-4 months, about 4-5 months, about 5-6 months, about 6-7 months, about 7-8 months, about 8-9 months, about 9-10 months, about 10-11 months, about 11-12 months, etc.

For some dosage forms, the SBEβCD may be present in an amount of about 1-200 mg; about 1-100 mg; 25-175 mg; about 50-150 mg; about 50-100 mg; about 50-200 mg; about 25-100 mg; about 75-150 mg; about 100-175 mg; about 20-80 mg; about 25-50 mg; about 60-100 mg; about 80-100 mg; about 100 mg; about 80-120 mg; about 100-120 mg; about 100-140 mg; about 120-160 mg; about 140-180 mg; about 150-200 mg, about 100-150 mg; about 30-90 mg; about 40-60 mg; about 40-80 mg; about 50-70 mg, about 55-65 mg, about 60-62 mg, or any amount in a range bounded by, or between, any of these values.

For some dosage forms, an inclusion complex of meloxicam and SBEβCD is about 1-10%, 5-20%, 5-15%, 6-16%, 7-17%, 8-18%, 9-19%, 10-20%, 15-30%, 30-40%, 40-50%, 50-70%, or 70-90% of the total weight of the dosage form, or any percentage in a range bounded by any of these values.

Dosage forms contain a bicarbonate (e.g., sodium bicarbonate) in amount of about 1-2000 mg; about 1-1000 mg; about 100-1000 mg; about 200-800 mg; about 1-500 mg; about 1-200 mg; about 1-100 mg; about 50-750 mg; about 500-1000 mg; about 100-500 mg; about 100-300 mg; about 500-1000 mg; about 300-700 mg; about 400-600 mg; about 50-250 mg; about 50-100 mg; about 250-750 mg; about 100-200 mg; about 200-300 mg; about 300-400 mg; about 400-500 mg; about 410-510 mg; about 420-520 mg; about 430-530 mg; about 440-540 mg; about 450-550 mg; about 460-560 mg; about 470-570 mg; about 480-580 mg; about 490-590 mg; about 500-600 mg; about 600-700 mg; about 700-800 mg; about 800-900 mg; about 900-1000 mg; about 150-650 mg; about 350-850 mg; about 400 mg; about 450 mg; about 500 mg, about 550 mg; about 600 mg; or any amount in a range bounded by, or between, any of these values.

A bicarbonate, such as sodium bicarbonate, may be at least about 10%, at least about 15%, at least about 20%, about 20-40%, about 30-50%, about 40-60%, about 50-70%, about 60-80%, or about 70-90%, or any percentage in a range bounded by any of these values, of the total weight of the dosage form.

The daily dose of meloxicam, or the amount of meloxicam administered in a single day (either in one administration, or by more than one divided doses adding up to the daily dose) may be given as a single dose, given once a day, or may be given in 2, 3, 4, or more divided doses during a day.

The weekly dose of meloxicam or the amount of meloxicam administered in a week (either in one administration, or by more than one divided doses adding up to the weekly dose) may be given as a single dose, given once a week, or may be given in 2, 3, 4, 5, 6, or 7 individual doses during a week.

The monthly dose of meloxicam (e.g., an oral dose), or a dose administered over a period of a month, may be given as a single dose, or as two or more individual doses administered during the month. The monthly dose may be administered bi-weekly in 2 or 3 divided doses. The monthly dose may be administered weekly in 4 or 5 divided doses. The monthly dose may be administered daily in 28 to 31 divided doses, or in 56 to 62 divided doses or more. The monthly dose may be administered in 5 to 15 individual doses during the month. The monthly dose may be administered for only 1 month, or may be repeatedly administered for 2, 3, 4, 5, 6, or more months.

The daily dose of rizatriptan may be e given as a single dose, given once a day, or may be given in 2, 3, 4, or more divided doses during a day.

The weekly dose of rizatriptan may be given as a single dose, given once a week, or may be given in 2, 3, 4, 5, 6, or 7 individual doses during a week.

The monthly dose of rizatriptan, or a total dose administered within a period of a month, may be given as a single dose, or as two or more individual doses administered during the month. The monthly dose may be administered bi-weekly in 2 or 3 divided doses. The monthly dose may be administered weekly in 4 or 5 divided doses. The monthly dose may be administered daily in 28 to 31 divided doses, or in 56 to 62 divided doses or more. The monthly dose may be administered in 5 to 15 individual doses during the month. The monthly dose may be administered for only 1 month, or may be repeatedly administered for 2, 3, 4, 5, 6, or more months.

The dosage form may be administered weekly for about one, two, three, four, or more consecutive weeks, every other week or bi-weekly, or once every three weeks. This regimen may be repeated once weekly, twice in a month, three times in a month, once monthly, once every two months, once every three months, or as directed by a medical professional.

Any reference to Tₘₐₓ, Cₘₐₓ, AUC, or any other pharmacokinetic parameter should be understood to include an average, mean, or median value in human beings, such as human patients or human subjects.

Unless otherwise indicated, the AUC refers to the AUC calculated to the last measured concentration (AUC₀₋ₜ), over a period of 24 hours (AUC₀₋₂₄), or extrapolated to infinity (AUC_{0-inf}).

For example, a dosage form described herein may reduce the Tₘₐₓ of meloxicam, such as a median, mean, or average Tₘₐₓ of meloxicam in human beings. In some embodiments, the dosage form may be used to treat a patient to achieve the Tₘₐₓ of meloxicam in the patient within about 10 minutes; within about 20 minutes; within about 30 minutes; within about 40 minutes; within about 50 minutes; within about 60 minutes; within about 70 minutes; within about 80 minutes; within about 90 minutes; within about 100 minutes; within about 110 minutes; within about 120 minutes; within about 180 minutes; about 10-30 minutes; about 20-40 minutes, about 30-50 minutes, about 40-60 minutes; about 50-70 minutes; about 60-90 minutes; about 70-100 minutes; about 80-110 minutes; about 90-120 minutes; about 1-10 hr; about 2-9 hr; about 3-7 hr; about 4-6 hr; about 1-5 hr; about 2-7 hr; about 3-8 hr; about 4-9 hr; about 1-4 hr; about 2-5 hr; about 3-6 hr; about 4-7 hr; about 5-8 hr; about 6-9 hr; about 7-10 hr; or any Tₘₐₓ in a range bounded by, or between, any of these values; after administration of the dosage forms described above.

An oral dosage form may have a Tₘₐₓ of meloxicam, such as a median, mean, or average Tₘₐₓ of meloxicam in human being, that is shorter than would be achieved by administering meloxicam by intramuscular injection. An oral dosage form may have a Tₘₐₓ of meloxicam that is shorter, or may increase meloxicam plasma levels at a faster rate, by a factor of at least about 1.5, about 2, about 3, about 4, about 5, about 6, about 7, about 8, about 9, about 10, about 12, about 15, about 20, or by a factor of about 1.1-2, about 1.5-3, about 2-4, about 3-5, about 4-6, about 1.5-1000, about 2-100, about 3-100, about 4-100, about 5-100, about 6-100, about 7-100, about 8-100, about 9-100, about 10-100, about 12-100, about 15-100, about 20-100, or by a factor in a range bounded by any of these values, as compared to that observed by intramuscular injection.

An oral dosage form may have a time to half-maximal plasma concentration of meloxicam, such as a median, mean, or average time to half-maximal plasma concentration in human beings, that is less than about 5 minutes; less than about 10 minutes; less than about 15 minutes; less than about 20 minutes; less than about 25 minutes; less than about 30 minutes; less than about 35 minutes; less than about 40 minutes; less than about 45 minutes; less than about 50 minutes; less than about 55 minutes; less than about 60 minutes; less than about 90 minutes; about 5-15 minutes; about 10-20 minutes, about 15-25 minutes, about 20-30 minutes; about 25-35 minutes; about 30-45 minutes; about 35-50 minutes; about 40-55 minutes; about 45-60 minutes; about 0.5-5 hours; or any time in a range bounded by any of these values.

The dosage form is formulated for oral administration, for example, with an inert diluent or with an edible carrier, or it may be enclosed in hard or soft shell gelatin capsules, compressed into tablets, or incorporated directly with the food of the diet. For oral therapeutic administration, the active compound may be incorporated with an excipient and used in the form of ingestible tablets, buccal tablets, coated tablets, troches, capsules, wafers, , and the like.

Tablets, troches, pills, capsules and the like may also contain one or more of the following: a binder such as gum tragacanth, acacia, corn starch or gelatin; an excipient, such as dicalcium phosphate; a disintegrating agent such as corn starch, potato starch, alginic acid and the like; a lubricant such as magnesium stearate; a sweetening agent such as sucrose, lactose or saccharin; or a flavoring agent such as peppermint, oil of wintergreen or cherry flavoring. When the unit dosage form is a capsule, it may contain, in addition to materials of the above type, a liquid carrier. Various other materials may be present as coating, for instance, tablets, pills, or capsules may be coated with shellac, sugar or both. It may be desirable for material in a dosage form or pharmaceutical composition to be pharmaceutically pure and substantially nontoxic in the amounts employed.

In addition to meloxicam, a SBEβCD, rizatriptan, and a bicarbonate, some dosage forms may contain excipients such as microcrystalline cellulose (e.g. about 1-20%), starch (e.g. about 1-10%), fumed silica (e.g. 0.1-10%), polyvinylpyrrolidone (e.g. about 1-10%), and/or magnesium stearate (e.g. about 0.1-10%).

The dosage form may further comprise an additional therapeutically active agents, such as an acid inhibitor or an analgesic.

The dosage form may further comprise an acid inhibitor present in an amount effective to raise the gastric pH of a patient to at least 2, to at least 2.5, to at least 3, to at least 3.5, to at least 4, and more to at least 5, when one or more unit dosage forms are administered. The term "acid inhibitor" refers to agents that inhibit gastric acid secretion and increase gastric pH. Specific H₂ blockers, also referred to as H₂ antagonists or histamine H₂ blockers or antagonists, which may be used include but are not limited to cimetidine, ranitidine, ebrotidine, pabutidine, lafutidine, loxtidine, famotidine, or combinations thereof.

Other agents that may be effectively used as acid inhibitors are the proton pump inhibitors such as omeprazole, esomeprazole, pantoprazole, lansoprazole, dexlansoprazole, rabeprazole, pariprazole, leminoprazole and tenatoprazole. The daily dose of the acid inhibitor, such as esomeprazole, may be about 1-200 mg, about 1-100 mg, about 50-100 mg, about 1-50 mg, about 40-80 mg, about 5-50 mg, about 20-40 mg, about 10-50 mg, about 10-20 mg, about 20-40 mg, about 15-50 mg, about 30-60 mg, about 10 mg, about 20 mg, about 30 mg, about 40 mg or any other amount in a range bounded by, or between, any of these values.

Examples of particular proton pump inhibitors include esomeprazole, present in unit dosage forms in an amount of between 5 mg and 50 mg; omeprazole, present in unit dosage forms in an amount of between 5 mg and 50 mg; lansoprazole, present in unit dosage forms in an amount of between 5 mg and 150 mg (and preferably at between 5 mg and 30 mg); and pantoprazole, present in unit dosage forms in an amount of between 10 mg and 200 mg. The proton pump inhibitor (such as esomeprazole) may be present in the dosage form in an amount of about 10-30 mg, about 20-40 mg, about 30-50 mg, about 40-60 mg, about 50-70 mg, about 60-80 mg, about 70-90 mg, or about 80-100 mg. Recently, a newer class of acid inhibitor has been developed which competes with potassium at the acid pump. The compounds in this class have been referred to as "reversible proton pump inhibitors" or "acid pump antagonists" and may also be used. Examples include AZD-0865, AR-H047108, CS-526, pumaprazole, revaprazan and soraprazan (see WO9605177 and WO9605199). Other compounds in this group are H-335/25 (AstraZeneca, Dialog file 128, accession number 020806); Sch-28080 (Schering Plough, Dialog file 128, accession number 009663); Sch-32651 (Schering Plough, Dialog file 128, accession number 006883) and SK&F-96067 (CAS Registry no. 115607-61-9).

Additional therapeutically active agents may include an analgesic such as a second non-steroidal anti-inflammatory drug, an opioid, a steroid, a triptan, etc. The dosage form or treatment may also further comprise administering a second non-steroidal anti-inflammatory drug in an amount effective to reduce or eliminate pain or inflammation. It will be understood that, for the purposes of the present disclosure, reference to an acid inhibitor, NSAID, or analgesic agent will include all of the common forms of these compounds and, in particular, their pharmaceutically acceptable salts. The amounts of NSAIDs which are therapeutically effective may be lower in the current disclosure than otherwise found in practice due to potential positive kinetic interaction and NSAID absorption in the presence of an acid inhibitor, and or in the presence of a buffering agent.

The dosage form or treatment may further comprise administering an opioid in an amount effective to reduce or eliminate pain or inflammation. The opioid may include, but is not limited to, (dextro)propoxyphene, A-methylfentanyl, alfentanil, allylprodine, bezitramide, buprenorphine, butorphanol, carfentanyl, desmethylprodine, dextromoramide, dezocine, diacetylmorphine, dihydrocodeinone, dihydroetorphine, dimorphone, diphenoxylate, dipipanone, etorphine, fentanyl, ketobemidone, lefetamine, levacetylmethadol, levomethorphan, levorphanol, loperamide, meperidine, meptazinol, methadone, methylmorphine, morphine, nalbuphine, nalmefene, naloxone, naltrexone, nicomorphine, ohmefentanyl, oripavine, oxycodone, oxymorphone, PEPAP, paramorphine, pentazocine, phenazocine, piritramide, prodine, remifentanil, sufentanil, tapentadol, tilidine, tramadol, or combinations thereof.

The term "unit dosage form" as used herein refers to a single entity for drug administration. A "unit dosage form" (or "unit dose form") may also be referred to as a "fixed dosage form" (or "fixed dose form") or "fixed dosage combination" (or "fixed dose combination") and are otherwise interchangeable. The unit dosage form may be a multilayer tablet.

The unit dosage form is suitable for oral administration to a patient. The unit dosage form may be a tablet. The unit dosage form may be a multilayer tablet comprising a single core and one or more layers outside of the core.

Some dosage forms may comprise a first layer comprising meloxicam, an SBEβCD, and a bicarbonate; and a second layer comprising a rizatriptan and a bicarbonate.

The first layer may contain, for example, any amount of meloxicam in one of the ranges recited above. For example, all of the meloxicam in the dosage form may be present in the first layer. The second layer may contain all of rizatriptan, such that any amount in the ranges recited above with respect to the triptan may apply to the second layer.

The first layer may contain about 10-200 mg, about 50-150 mg, about 50-100 mg, about 70-120 mg, about 90-140 mg, or about 100 mg of the bicarbonate, such as sodium bicarbonate, or any amount of the bicarbonate in a range bounded by any of these values.

The second layer may contain about 100-500 mg, about 200-500 mg, about 300-500 mg, about 350-450 mg, about 380-420 mg, or about 400 mg of the bicarbonate, such as sodium bicarbonate, or any amount of the bicarbonate in a range bounded by any of these values.

Some oral dosage forms may have enteric coatings or film coatings. A dosage form may comprise a tablet or a capsule having an enteric coating. A dosage form may comprise a tablet or a capsule having a film coating.

### Example 1

The effect of varying amounts of potassium carbonate (K₂CO₃) and sodium bicarbonate (NaHCO₃) on the pH of acidic media was tested. The acidic media was chosen to simulate gastric conditions. K₂CO₃ or NaHCO₃ was added to 50 mL of a 0.01 N HCl solution (pH 2). The pH of the solution was measured after addition of the K₂CO₃ or NaHCO3. Deionized water (240 mL) was then added to the mixture and pH was measured again. The results are shown in Tables 1-4.

**Table 1. Results with K₂CO₃ (0.01 N HCl)**

| K₂CO₃ (mg) | pH |
|---|---|
| 25 | 2.84 |
| 35 | 6.29 |
| 45 | 8.05 |
| 50 | 8.29 |
| 100 | 9.43 |
| 200 | 10.14 |
| 300 | 10.39 |
| 400 | 10.55 |
| 450 | 10.58 |

**Table 2. Results with K₂CO₃ (0.01 N HCl + Water)**

| K₂CO₃ (mg) | pH |
|---|---|
| 200 | 10.27 |
| 300 | 10.46 |
| 400 | 10.57 |
| 450 | 10.63 |

**Table 3. Results with NaHCO₃ (0.01 N HCl)**

| NaHCO₃ (mg) | pH |
|---|---|
| 200 | 5.28 |
| 300 | 5.90 |
| 400 | 6.44 |
| 450 | 6.86 |
| 500 | 8.23 |
| 750 | 8.30 |
| 1000 | 8.36 |

**Table 4. Results with NaHCO₃ (0.01 N HCl + Water)**

| NaHCO₃ (mg) | pH |
|---|---|
| 200 | 5.41 |
| 300 | 5.89 |
| 400 | 6.11 |
| 450 | 6.46 |
| 500 | 8.33 |
| 750 | 8.54 |
| 1000 | 8.60 |

### Example 2

Tablets containing meloxicam and combinations of cyclodextrin, K₂CO3, or NaHCO₃ were manufactured and tested for dissolution. Tablets containing meloxicam alone (MOBIC^{®}) were purchased and also tested for dissolution. The tested tablets are listed in Table 5. Meloxicam in the form of meloxicam/cyclodextrin inclusion complexes was used in the tablets containing meloxicam and cyclodextrin. The inclusion complexes were formed by mixing meloxicam and cyclodextrin in an aqueous pH-adjusted solution. The pH of the solution was adjusted using buffering agents. The resulting soluble meloxicam/cyclodextrin inclusion complexes were then spray dried. This spray-dried dispersion was used in the manufacture of the tablets containing cyclodextrin.

**Table 5. Tablets**

| | |
|---|---|
| Tablet A | 15 mg meloxicam + 25 mg K₂CO3 |
| Tablet B | 15 mg meloxicam + 50 mg K₂CO3 |
| Tablet C | 15 mg meloxicam + 100 mg K₂CO3 |
| Tablet D | 15 mg meloxicam + 150 mg K₂CO3 |
| Tablet E | 15 mg meloxicam + 500 mg NaHCO3 |
| Tablet F | 15 mg meloxicam + 100 mg SBEβCD |
| Tablet G | 15 mg meloxicam + 100 mg SBEβCD + 25 mg K₂CO3 |
| Tablet H | 15 mg meloxicam + 100 mg SBEβCD + 50 mg K₂CO3 |
| Tablet I | 15 mg meloxicam + 100 mg SBEβCD + 100 mg K₂CO3 |
| Tablet J | 15 mg meloxicam + 100 mg SBEβCD + 150 mg K₂CO3 |
| Tablet K | 15 mg meloxicam + 100 mg SBEβCD + 500 mg NaHCO3 |
| Tablet L | 15 mg meloxicam (MOBIC^{®}) |

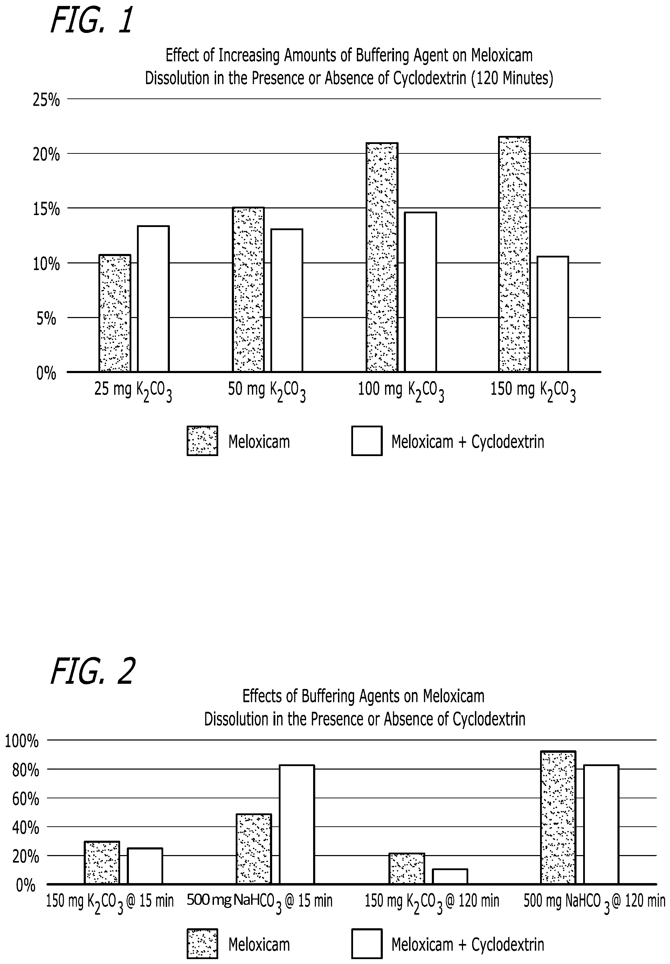

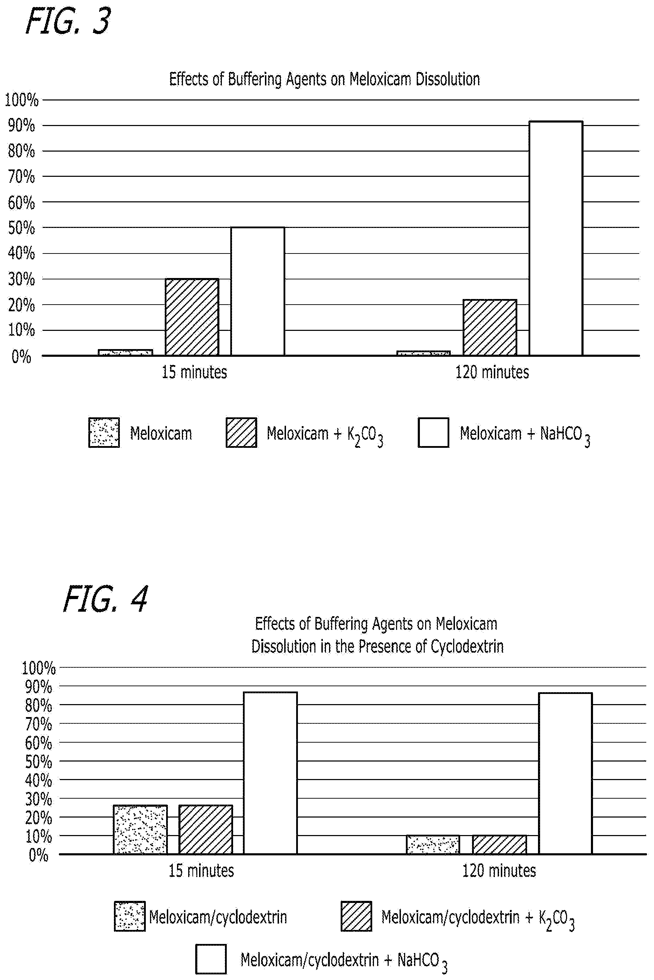

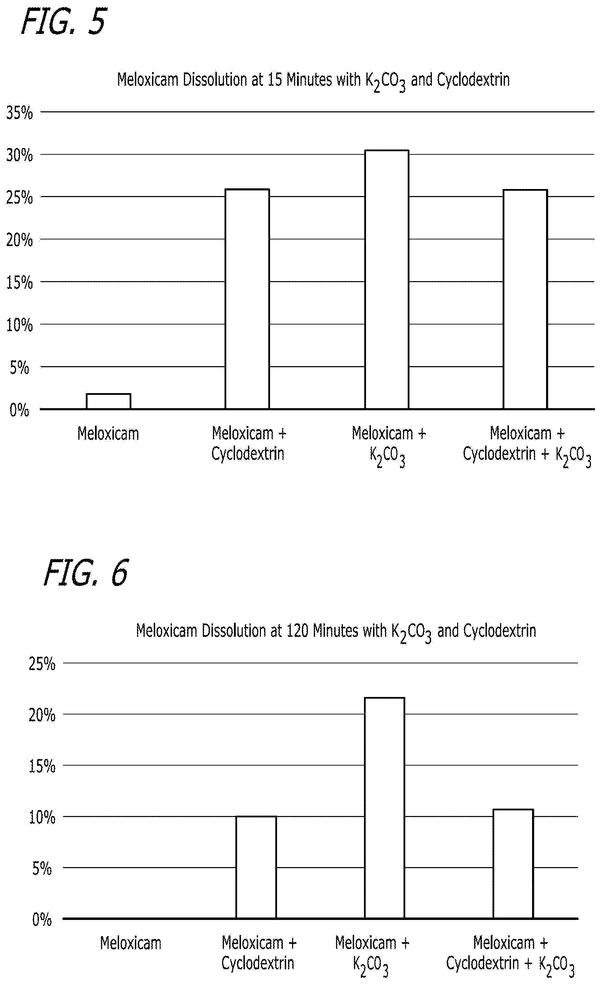

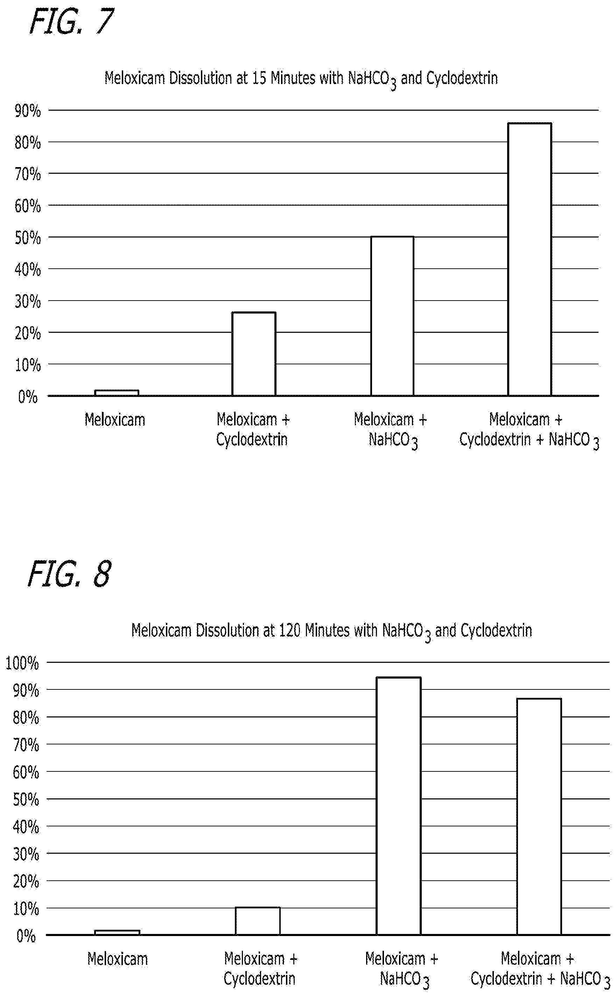

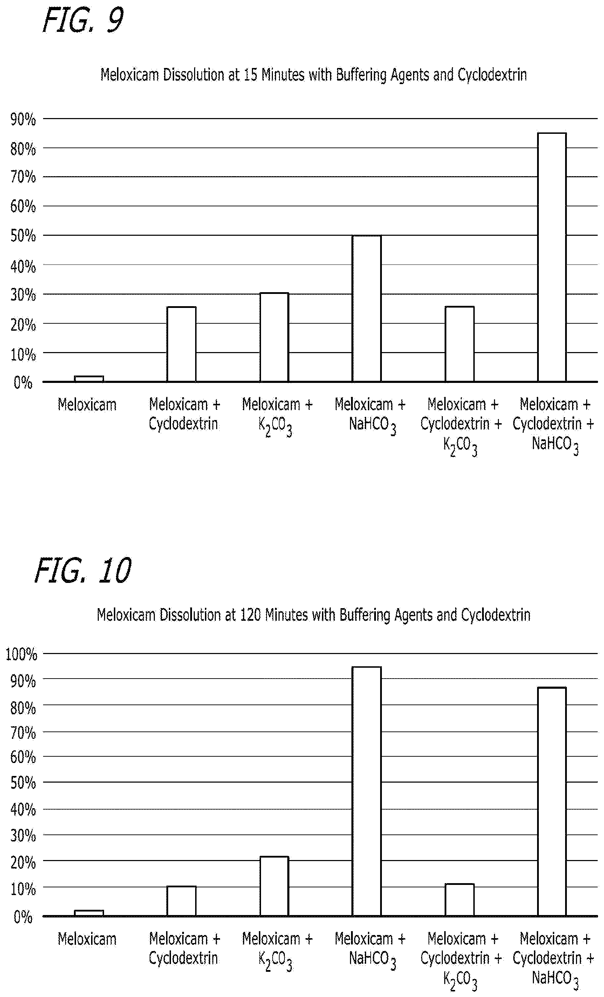

Dissolution testing in acidic medium (chosen to simulate gastric conditions) was performed by placing the tablets in a 0.01 N HCl solution, at an agitation rate of 75 RPM, and vessel temperature of approximately 37 °C. The results are presented in Tables 6 and in Figures 1-10. Results at various time points (0, 15, 30, 45, 60, 90, and 120 minutes) are presented as percent (%) of meloxicam dissolved.

**Table 6. Dissolution Results**

| | 0 mins | 15 mins | 30 mins | 45 mins | 60 mins | 90 mins | 120 mins |
|---|---|---|---|---|---|---|---|
| Tablet A | 0% | 23% | 17% | 15% | 13% | 12% | 11% |
| Tablet B | 0% | 27% | 20% | 17% | 16% | 17% | 15% |
| Tablet C | 0% | 31% | 26% | 25% | 24% | 23% | 21% |
| Tablet D | 0% | 30% | 26% | 25% | 24% | 23% | 22% |
| Tablet E | 0% | 50% | 66% | 77% | 84% | 92% | 95% |
| Tablet F | 0% | 26% | 17% | 14% | 12% | 11% | 10% |
| Tablet G | 0% | 48% | 39% | 26% | 20% | 16% | 14% |
| Tablet H | 0% | 44% | 30% | 22% | 17% | 16% | 13% |
| Tablet I | 0% | 32% | 33% | 27% | 21% | 16% | 15% |
| Tablet J | 0% | 26% | 27% | 19% | 15% | 12% | 11% |
| Tablet K | 0% | 85% | 86% | 86% | 86% | 86% | 86% |
| Tablet L | 0% | 2% | 2% | 2% | 2% | 2% | 2% |

Dissolution of meloxicam was greater with the tablets containing various combinations of meloxicam and cyclodextrin, K₂CO₃, or NaHCO₃, as compared to tablets containing meloxicam alone. For example, after 120 minutes, dissolution of meloxicam tablets containing NaHCO₃ was 95% as compared to 2% for tablets containing meloxicam alone.

Dissolution of meloxicam increases with increasing amounts of K₂CO₃ in the absence of cyclodextrin. However, in the presence of cyclodextrin, increasing amounts of K₂CO₃ did not appear to increase meloxicam dissolution. At the highest dose of potassium carbonate tested, meloxicam dissolution in the presence of cyclodextrin was reduced by approximately 50% as compared to meloxicam dissolution in the absence of cyclodextrin at 120 minutes.

Dissolution of meloxicam with NaHCO₃ was significantly greater than that observed with the highest dose of K₂CO₃ at 15 minutes (50% versus 30%), and at 120 minutes (92% versus 23%). Meloxicam dissolution in the presence of cyclodextrin was also significantly greater with NaHCO₃ as compared to the highest dose of K₂CO₃ at 15 minutes (85% versus 26%), and at 120 minutes (86% versus 12%). NaHCO₃ in the presence of cyclodextrin increased meloxicam dissolution at 15 minutes as compared to potassium bicarbonate which resulted in a reduction in dissolution.

### Example 3

A bilayer tablet containing 1) an inclusion complex of SBEβCD with meloxicam prepared as described in Example 2, and 2) sodium bicarbonate was prepared (SBEβCD-Meloxicam/Bicarbonate). The first layer contained an inclusion complex of 15 mg meloxicam and 100 mg SBEβCD, and 100 mg of sodium bicarbonate. The second layer contained 40 mg of esomeprazole and 400 mg of sodium bicarbonate.

A total of 20 human subjects were randomly assigned in a 1:1 ratio to treatment with the SBEβCD-Meloxicam/Bicarbonate tablets described above or Mobic^{®} tablets (15 mg meloxicam), once daily for 6 days under fasting conditions.

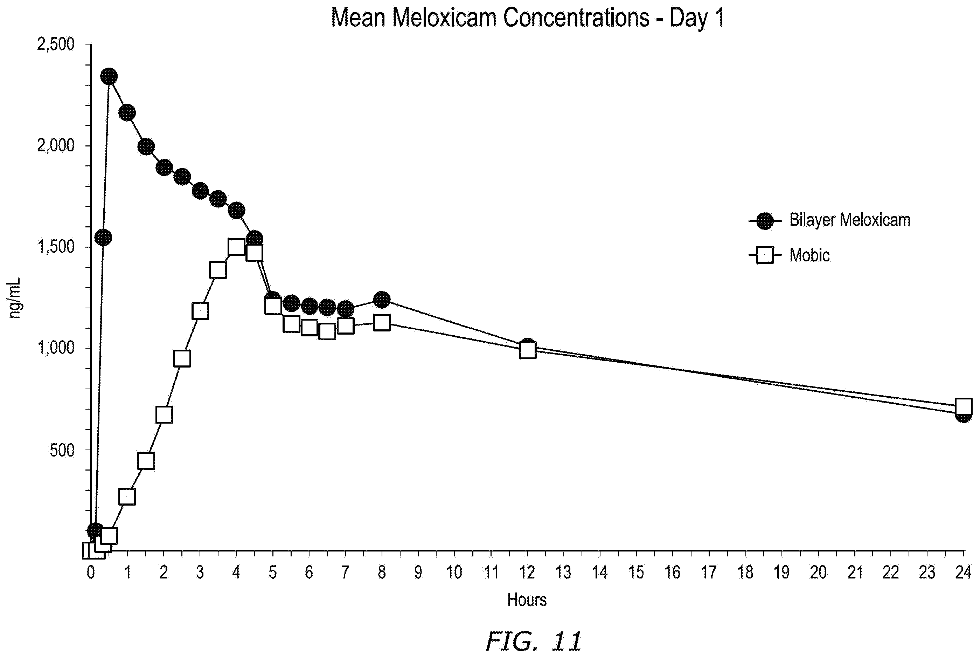

On the first day of dosing, plasma samples were collected for concentration analysis of meloxicam at several time points. Concentrations of meloxicam were determined using LC-MS/MS. Pharmacokinetic parameters were calculated. The results are depicted in FIG. 11.

The median Tₘₐₓ for meloxicam, the trial's primary endpoint, was 9 times faster for the SBEβCD-Meloxicam/Bicarbonate tablets as compared to Mobic^{®} (0.5 hour versus 4.5 hours respectively, p<0.0001).

The SBEβCD-Meloxicam/Bicarbonate tablets also demonstrated higher mean maximum plasma concentration (Cₘₐₓ) (p=0.0018), faster time to therapeutic plasma concentration (p<0.0001), and faster time to half-maximal plasma concentration (p<0.0001) as compared to Mobic^{®}.

### Example 4

A monolayer tablet containing 1) the inclusion complex of SBEβCD with meloxicam; 2) rizatriptan; and 3) sodium bicarbonate was prepared (SBEβCD-Meloxicam/rizatriptan/Bicarbonate). The monolayer tablet contained 20 mg of meloxicam, 10 mg of rizatriptan, and 500 mg of sodium bicarbonate. The inclusion complex was the same as the inclusion complex of Example 3.

Dissolution testing of the tablets in acidic medium (chosen to simulate gastric conditions) was performed by placing the tablets in a 0.01 N HCl solution, at an agitation rate of 75 RPM, and vessel temperature of approximately 37 °C. The results are presented in Tables 7. Results at various time points (0, 15, 30, 45, 60, 90, and 120 minutes) are presented as percent (%) of meloxicam, and percent (%) of rizatriptan dissolved.

**Table 7. Dissolution Results**

| **Time-point (minutes)** | **0 min** | **15 min** | **30 min** | **45 min** | **60 min** | **90 min** | **120 min** |
|---|---|---|---|---|---|---|---|
| Rizatriptan | 0% | 89% | 102% | 103% | 103% | 103% | 103% |
| Meloxicam | 0% | 79% | 92% | 93% | 93% | 93% | 94% |

As shown in Table 7, the dissolution results of the tablets in Example 4 are very similar to the dissolution result of Example 2. Therefore, we expect the pharmacokinetic properties, including bioavailability, Tₘₐₓ, etc., of the tablets in Example 4 to be similar to those described in Example 3 and FIG. 11.

Unless otherwise indicated, all numbers expressing quantities of ingredients, properties such as molecular weight, reaction conditions, and so forth used in the specification and claims are to be understood in all instances as indicating both the exact values as shown and as being modified by the term "about." Accordingly, unless indicated to the contrary, the numerical parameters set forth in the specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

The terms "a," "an," "the" and similar referents used in the context of describing the invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of any claim. No language in the specification should be construed as indicating any non-claimed element essential to the practice of the invention.

## Claims

1. A solid oral dosage form comprising:
an inclusion complex of meloxicam and a sulfobutyl ether β-cyclodextrin (SBEβCD), wherein the solid dosage form contains about 20 mg of the meloxicam or a molar equivalent amount of a salt thereof;
a bicarbonate; and
about 10 mg of rizatriptan or a molar equivalent amount of a salt thereof.

2. The solid oral dosage form of claim 1 having a shorter Tₘₐₓ of meloxicam in a mammal than a reference dosage form that:
1) contains the same amount of the meloxicam;
2) does not contain an SBEβCD; and
3) does not contain a bicarbonate,
when directly administered orally to the mammal.

3. The solid oral dosage form of claim 1, comprising an inclusion complex of:
1) the rizatriptan; and
2) the SBEβCD.

4. The solid oral dosage form of any one of Claims 1 to 3, wherein the SBEβCD has 6 to 7 sulfobutyl ether groups for each molecule of β-cyclodextrin.

5. The solid oral dosage form of any preceding claim, containing 50 mg to 200 mg of the SBEβCD.

6. The solid oral dosage form of claim 5, containing about 100 mg of the SBEβCD.

7. The solid oral dosage form of any preceding claim, wherein the bicarbonate comprises sodium bicarbonate.

8. The solid oral dosage form of any one of claims 1 to 6, containing 400 mg to 600 mg of the bicarbonate.

9. The solid oral dosage form of claim 7, containing about 500 mg of sodium bicarbonate.

10. The solid oral dosage form of claim 9, wherein the solid oral dosage form has been shown to have a mean Tₘₐₓ of meloxicam that is less than about 2 hours.

11. The solid oral dosage form of any preceding claim, wherein the solid oral dosage form has increased bioavailability of the meloxicam as compared to the reference dosage form defined in claim 2 when administered to a mammal.

12. The solid oral dosage form of any preceding claim, wherein the solid oral dosage form has increased bioavailability of the rizatriptan as compared to the reference dosage form defined in claim 2 when administered to a mammal.

13. The solid oral dosage form according to any one of claims 1 to 12 for use in the treatment of pain in a mammal in need thereof.

14. The solid oral dosage form for use according to claim 13,wherein the pain is migraine.

15. The solid oral dosage form for use according to claim 13, wherein the pain is inflammatory pain.

## Patentansprüche

1. Eine feste, orale Dosierungsform, beinhaltend:
einen Inklusionskomplex von Meloxicam und ein Sulfobutylether-β-Cyclodextrin (SBEβCD), wobei die feste Dosierungsform etwa 20 mg des Meloxicams oder eine molar äquivalente Menge eines Salzes davon enthält;
ein Bicarbonat; und
etwa 10 mg Rizatriptan oder eine molar äquivalente Menge eines Salzes davon.

2. Feste, orale Dosierungsform gemäß Anspruch 1 mit einer kürzeren Tₘₐₓ von Meloxicam bei einem Säugetier als eine Referenz-Dosierungsform, die:
1) die gleiche Menge des Meloxicams enthält;
2) kein SBEβCD enthält; und
3) kein Bicarbonat enthält,
wenn dem Säugetier direkt oral verabreicht.

3. Feste, orale Dosierungsform gemäß Anspruch 1, beinhaltend einen Inklusionskomplex von:
1) dem Rizatriptan; und
2) dem SBEβCD.

4. Feste, orale Dosierungsform gemäß einem der Ansprüche 1 bis 3, wobei das SBEβCD 6 bis 7 Sulfobutylethergruppen für jedes Molekül an β-Cyclodextrin aufweist.

5. Feste, orale Dosierungsform gemäß einem vorhergehenden Anspruch, die 50 mg bis 200 mg des SBEßCDs enthält.

6. Feste, orale Dosierungsform gemäß Anspruch 5, die etwa 100 mg des SBEßCDs enthält.

7. Feste, orale Dosierungsform gemäß einem vorhergehenden Anspruch, wobei das Bicarbonat Natriumbicarbonat beinhaltet.

8. Feste, orale Dosierungsform gemäß einem der Ansprüche 1 bis 6, die 400 mg bis 600 mg des Bicarbonats enthält.

9. Feste, orale Dosierungsform gemäß Anspruch 7, die etwa 500 mg an Natriumbicarbonat enthält.

10. Feste, orale Dosierungsform gemäß Anspruch 9, wobei von der festen, oralen Dosierungsform gezeigt wurde, dass sie eine mittlere Tₘₐₓ von Meloxicam aufweist, die weniger als etwa 2 Stunden beträgt.

11. Feste, orale Dosierungsform gemäß einem vorhergehenden Anspruch, wobei die feste, orale Dosierungsform im Vergleich mit der in Anspruch 2 definierten Referenz-Dosierungsform eine gesteigerte Bioverfügbarkeit des Meloxicams aufweist, wenn einem Säugetier verabreicht.

12. Feste, orale Dosierungsform gemäß einem vorhergehenden Anspruch, wobei die feste, orale Dosierungsform im Vergleich mit der in Anspruch 2 definierten Referenz-Dosierungsform eine gesteigerte Bioverfügbarkeit des Rizatriptans aufweist, wenn einem Säugetier verabreicht.

13. Feste, orale Dosierungsform gemäß einem der Ansprüche 1 bis 12 zur Verwendung bei der Behandlung von Schmerzen bei einem Säugetier mit Bedarf daran.

14. Feste, orale Dosierungsform zur Verwendung gemäß Anspruch 13, wobei die Schmerzen Migräne sind.

15. Feste, orale Dosierungsform zur Verwendung gemäß Anspruch 13, wobei die Schmerzen entzündliche Schmerzen sind.

## Revendications

1. Une forme galénique orale solide comprenant :
un complexe d'inclusion de méloxicam et d'une β-cyclodextrine d'éther sulfobutylique (SBEβCD), où la forme galénique solide contient environ 20 mg du méloxicam ou une quantité molaire équivalente d'un sel de celui-ci ;
un bicarbonate ; et
environ 10 mg de rizatriptan ou une quantité molaire équivalente d'un sel de celui-ci.

2. La forme galénique orale solide de la revendication 1 ayant un Tₘₐₓ de méloxicam plus court chez un mammifère qu'une forme galénique de référence qui :
1) contient la même quantité du méloxicam ;
2) ne contient pas de SBEβCD ; et
3) ne contient pas de bicarbonate,
quand elle est administrée directement par voie orale au mammifère.

3. La forme galénique orale solide de la revendication 1, comprenant un complexe d'inclusion :
1) du rizatriptan ; et
2) de la SBEβCD.

4. La forme galénique orale solide de l'une quelconque des revendications 1 à 3, où la SBEβCD a de 6 à 7 groupes éther sulfobutylique pour chaque molécule de β-cyclodextrine.

5. La forme galénique orale solide de n'importe quelle revendication précédente, contenant de 50 mg à 200 mg de la SBEβCD.

6. La forme galénique orale solide de la revendication 5, contenant environ 100 mg de la SBEβCD.

7. La forme galénique orale solide de n'importe quelle revendication précédente, où le bicarbonate comprend le bicarbonate de sodium.

8. La forme galénique orale solide de l'une quelconque des revendications 1 à 6, contenant de 400 mg à 600 mg du bicarbonate.

9. La forme galénique orale solide de la revendication 7, contenant environ 500 mg de bicarbonate de sodium.

10. La forme galénique orale solide de la revendication 9, la forme galénique orale solide ayant démontré avoir un Tₘₐₓ moyen de méloxicam qui est inférieur à environ 2 heures.

11. La forme galénique orale solide de n'importe quelle revendication précédente, la forme galénique orale solide ayant une biodisponibilité du méloxicam accrue comparée à la forme galénique de référence définie à la revendication 2 quand elle est administrée à un mammifère.

12. La forme galénique orale solide de n'importe quelle revendication précédente, la forme galénique orale solide ayant une biodisponibilité du rizatriptan accrue comparée à la forme galénique de référence définie à la revendication 2 quand elle est administrée à un mammifère.

13. La forme galénique orale solide selon l'une quelconque des revendications 1 à 12 pour son utilisation dans le traitement d'une douleur chez un mammifère en ayant besoin.

14. La forme galénique orale solide pour l'utilisation selon la revendication 13, où la douleur est une migraine.

15. La forme galénique orale solide pour l'utilisation selon la revendication 13, où la douleur est une douleur inflammatoire.
